# EUROPEAN PATENT SPECIFICATION

(11) **EP 3 339 856 B1**
(45) Date of publication and mention of the grant of the patent: **28.11.2018**
(21) Application number: 16002718.1
(22) Date of filing: 22.12.2016
(51) Int. Cl.: G01N 33/18

(54) **METHOD FOR ETHOLOGICAL MONITORING OF CRAYFISH AND SYSTEM FOR THIS METHOD**
VERFAHREN ZUR ETHOLOGISCHEN ÜBERWACHUNG VON FLUSSKREBS UND SYSTEM FÜR DIESES VERFAHREN
PROCÉDÉ DE SURVEILLANCE ÉTHOLOGIQUE D'ÉCREVISSES ET SYSTÈME POUR LEDIT PROCÉDÉ

(43) Date of publication of application: 27.06.2018
(73) Proprietor: Jihoceska Universita V Ceskych Budejovicich, 389 25 Vodnany (CZ)
(72) Inventor: Cisar, Petr, 378 10 Ceske Velenice (CZ); Kozak, Pavel, 398 11 Protivin (CZ); Soucek, Pavel, 378 16 Lomnice nad Luznici (CZ); Pautsina, Aliaksandr, 370 04 Ceské Budejovice (CZ)
(74) Representative: Sedlák, Jirí

(56) References cited:
- CZ-B6- 305 212
- KR-A- 20110 121 863
- IRYNA KUKLINA ET AL: "Real-time monitoring of water quality using fish and crayfish as bio-indicators: a review", ENVIRONMENTAL MONITORING AND ASSESSMENT, vol. 185, no. 6, 7 October 2012 (2012-10-07), pages 5043-5053, XP055376003, NL ISSN: 0167-6369, DOI: 10.1007/s10661-012-2924-2
- P. KOZÁK ET AL: "Stress reaction in crayfish: chlorides help to withstand stress in high nitrite concentration conditions - preliminary study", KNOWLEDGE AND MANAGEMENT OF AQUATIC ECOSYSTEMS, no. 401, 1 January 2011 (2011-01-01), page 05, XP055376615, DOI: 10.1051/kmae/2011014

## Description

### Field of the invention

The present invention relates the area of bio-indicators of the quality of the environment, particularly a method for the ethological monitoring of crayfish in an aquarium and a system designed to implement this method.

### Background of the invention

The utilization of bio-indicators of the quality of the environment is one of the available methods of the detection of changes caused by various pollutants which currently ranks among the mainstream eco-biological research. The main advantage of the use of organisms as bio-indicators is the possibility of fast detection of also low concentrations of the pollutant in the environment subject to monitoring due to the high sensitivity and rapid reaction of the used organism, namely in the order of seconds. On the other hand, it is extremely important to treat the selected group of organisms concerned properly and try to cause minimal stress and provide the organism with as natural conditions as possible also during monitoring in laboratory conditions. The organism utilised as a bio-indicator is placed into its common environment where it is monitored on a continuous basis. The organism's behaviour patterns are continuously monitored and subsequently recorded. This is the way how to detect possible reactions of the monitored organism to the pollution of its environment. The crustaceans taxon, especially crayfish, whose monitoring brought particularly good results, seems to be the best organisms for the monitoring of pollution of the aquatic environment. The advantage of the crustaceans is their great biodiversity, which means that for each environment a corresponding species can be found. Crayfish has a simple cardiovascular system, easy-to-monitor transmissions in the nervous system, exoskeleton and it can be easily bred in laboratory conditions.

For the monitoring of crayfish movement, for example in an aquarium or another water tank, a camera detecting the visible spectrum of light is used in some cases. The disadvantage of such a simple observation of the crayfish movement is the fact that crayfish is active at night, which compromises the quality of the camera recordings. To surmount this disadvantage, diodes emitting red light were attached to the crayfish carapace or crayfish were monitored by a camera the housing of which was equipped with polarized, partly linear lights. Unfortunately crayfish were disturbed by the artificial light so that their movements and heart beats were not natural and the observation results provided distorted data.

The cameras are put either above or under the aquarium where the monitored crayfish lives. For more accurate recording of the movement patterns of the observed crayfish, an instrument comprising a travelling table with a rotary board with an attached arm and a camera fixed to the arm's end was designed. This instrument allows the crayfish movements to be copied better which results in better and more accurate recordings. Nevertheless, a mere observation of the crayfish movements is not a sufficient indicator of a change in the environment due to pollution as it only allows a visual check of the crayfish behaviour, which can be very imprecise.

The detection of pollutants in water through crayfish is based on the correlation between the crayfish cardiac activity and the quantity of the pollutant in water. A number of systems to measure crayfish cardiac activity, heart rate have been invented. All systems invented to date use a sensor placed on the crayfish carapaces, which represents the first group of systems called non-invasive bio-monitoring. This method is represented e.g. by plethysmography which is based on the transmission of light beams through the tissue of the scanned area. A pulse plethysmographic sensor consists of two diodes emitting low-intensity light and a phototransistor. To be able to measure the crustaceans cardiac activity, the sensor must be attached externally onto the carapace above the heart using a water-resistant biodegradable adhesive. The low-intensity radiation light emitted by the diodes passes through the crayfish carapace into its pericardium. When the heart muscle contracts, the pericardium reflects part of the light which reaches the phototransistor which converts its light energy into a proportional change in voltage which is then transmitted to a processing device. The disadvantage of this instrument lies in its complicated setting and the subsequent evaluation of acquired data. This arrangement is described also in the patent CZ 305212 where the physiological function of crayfish together with their natural behaviour is monitored by a camera acquiring video recordings of crayfish which are then transmitted to a computer where they are compared and evaluated together with a digital signal from a non-invasive sensor designed to monitor physiological functions using common evaluation software. The non-invasive sensor is equipped with a source of infra-red light emitted into the crayfish body, and a receiving unit that receives the light reflected from the crayfish heart. The disadvantage of this arrangement lies in particular in the necessity to place the sensor on the crayfish carapace from where recordings are transmitted to the computer. The attached sensor and cable make the crayfish movement more difficult and create unnatural conditions resulting in stress which affects the crayfish cardiac activity.

The other group of systems called invasive bio-monitoring uses electrodes implanted into the crayfish body which measure a change in voltage. Among common disadvantages of these two general arrangements is the necessity to connect the sensor to the processing device usually by optical fibres, cables or other conductors. The sensor and conductors are an unnaturally limiting factor for the crayfish movement and may stress the crayfish during the experiment. The correlation between stressing factors and cardiac activity has been proven and experimentally determined and may hide changes in cardiac activity caused by pollutants present in water. Among these monitoring methods we can find for example impedance pneumography (IPG) that monitors respiratory movements. Using the impedance pneumograph, a low oscillating current (2 µA) is transmitted between small conductors that play the role of electrodes. Another method for invasive bio-monitoring is electrocardiography (ECG). Two insulated stainless steel wires are placed under the dorsal carapace directly above the heart. The wires are placed into holes drilled in the carapace and cemented by a quick-setting cyanoacrylate adhesive. All cardiac activity is recorded by an impedance detector that measures dynamic resistance between the stainless steel wires and transmitted to a computer. However, the aforementioned arrangements may significantly influence the resulting measured values and the conclusions thus become invalid.

The purpose of the invention is to create a method for the ethological monitoring of crayfish and also to develop a system to implement such a method while removing the aforementioned deficiencies. In particular, the invention should not restrict the movement of crayfish by the hardware components of the system and should cause no stress affecting their cardiac activity so that the changes in cardiac activity would only reflect the presence of undesirable pollutants in water subject to monitoring, or the crayfish movements.

### Summary of the invention

The above-mentioned disadvantages are removed by the real-time ethological monitoring of crayfish in water according to the present invention. With this method the crayfish placed in an aquarium is exposed to infra-red radiation from a source designed for the monitoring of cardiac activity while the crayfish movement is monitored by a camera. The measured data is transmitted to a computer in the form of a digital signal which is processed by a software module for the evaluation of the crayfish reactions to pollutants present in water.

The invention is based on the fact that the crayfish carapace is first equipped with at least two reflective markers whose mutual distance delimits the heart area of the crayfish and the infra-red light emitted from a distant source is directed at this area. At the same time, a distant infra-red radiation detector receives, on a wireless basis, the infra-red light reflected from the reflective markers and the infra-red light reflected from the crayfish pericardium within the delimited heart area. The acquired data of the beams is transmitted in the form of a video recording to the computer where the position and directional orientation of the crayfish is determined together with the position and directional orientation of the delimited heart area, heart rate and a trajectory of the crayfish movement using a software module on a real-time basis. The follow-up analysis of such data serves for the automatic determination of the physiological condition and changes in the quality of water or for the determination of the presence of chemical substances in water. The contactless monitoring of crayfish in an aquarium eliminates stress to which crayfish are exposed in water that is caused among other things by the restrained mobility of the crayfish. The time necessary for the adaptation of the crayfish to the environment containing fibres or conductors attached to its carapace, or where applicable, to the sensor placed on the crayfish carapace and the conductors leading from the sensor, is eliminated. In addition, the robustness of the solution under real conditions is increased by the elimination of cables and conductors used for the monitoring of the crayfish cardiac activity. Besides, heart beat is a very reliable indicator which is utilized in a majority of ethological studies and systems. The method for the ethological monitoring of crayfish is possible not only in crayfish who are resting in the stationary position, but also in crayfish who is moving freely.

The advantages of the use of crayfish for the purposes of this invention rest in the fact that crayfish have a very simple cardiovascular system allowing the crayfish heart beat to be monitored and evaluated easily. The intensity of the infra-red light reflected from the crayfish pericardium is summed up for all points of the heart area. The totalled intensity represents the cardiac response of the crayfish which is used for the determination of pulse rate per minute. The response signal is filtered using a noise reduction method. Using methods for the detection of local extremes in signal, local minimums and maximums corresponding to the heart muscle contractions are determined. Based on the determination of the number of extremes per minute, the crayfish heart activity frequency is determined. Heart beat and information on the crayfish position based on this invention are utilized for the analysis of the quality of water with the use of bio-indicators without exposure to stressful conditions considering the wireless design of the system.

In the preferred embodiment, an infra-red camera is used as a distant source and also a distant receiver of infra-red radiation. The main comparative advantage of this invention is the contactless measurement of the crayfish heart beat with the simultaneous detection of its position using one infra-red camera providing for transmission and also reception of infra-red radiation without the necessity to install any cables, conductors or optical fibres restricting the natural movement of crayfish.

The infra-red camera is preferably placed at a distance ranging from 400 to 1,500 mm from the bottom of the aquarium. Therefore, the system is able to monitor the heart beat of crayfish in the aquarium at a distance of tens of centimetres. Neither the infra-red camera, nor any of its components interfere with the natural environment of crayfish, i.e. the aquarium, and thus cause no stress to the crayfish being monitored.

In the preferred embodiment, the video recording is acquired by an infra-red camera with the minimum frequency of 30 images per second. The infra-red light that the camera emits into the crayfish body is not perceived by the crayfish so that the monitoring results are neither affected nor distorted.

The reflective markers are preferably attached to the carapace of the crayfish belonging to invasive species: *Pacifastacus leniusculus* and *Procambarus fallax f. virginalis.* The advantage of the various species of crayfish rests in the fact that they survive well in laboratory conditions, show a high degree of biodiversity, which means that naturally living crayfish can be found in various environments, which allow a high precision of the conducted measurements to be attained.

The invention also comprises the system to implement the method for the ethological monitoring of crayfish in an aquarium that is described above. The invention is based on the fact that at least one crayfish is equipped with two reflective markers attached to its carapace where the mutual distance of the reflective markers delimits the heart area of the crayfish. In addition, the system comprises at least one distant source of infra-red radiation for the directional emitting of infra-red radiation beams at the delimited heart area and at the reflective markers, and at least one distant receiver of infra-red radiation for wireless reception of the infra-red radiation beams reflected from the reflective markers and the infra-red radiation beams reflected from the crayfish pericardium within the delimited heart area. The system also comprises at least one computer for the reception of digitized data of the beams in the form of a video recording, which is equipped with a software module for the detection of the crayfish position and the directional orientation of the delimited heart area, heart beat and the trajectory of the crayfish movement in real time.

The wireless system for the real-time ethological monitoring of crayfish in an aquarium based on this invention provides for the automatic analysis of the quality of water with the use of bio-indicators without exposing them to stressful conditions. Another advantage of the system is the fact that it eliminates the necessity of its replacement due to the growth of the crayfish carapace as it is in the case of the contact sensor whose size must be adapted to the size of the crayfish carapace. The system also allows several crayfish to be monitored in one aquarium with the possibility of their identification, delimitation of their heart areas, determination of their heart beats and the trajectory of their movements in real time, which is a huge advantage. The previously known solutions did not allow this arrangement as there was a risk of the entanglement of the used fibres or conductors, which again contributed to the aggravation of stressful conditions for the crayfish and distortion of acquired data.

In the preferred embodiment, the distant source and receiver of infra-red radiation is an infra-red camera which is arranged above the aquarium at a distance ranging from 400 mm to 1,500 mm from the aquarium bottom.

The infra-red camera is preferably used with a source of infra-red radiation emitting wavelengths raging from 827 to 850 mm with the power of 60 mW. This covers the band of the spectrum that covers the infra-red radiation beams reflected from the crayfish pericardium. In a preferred embodiment, the reflective marker is attached to the carapace of the crayfish belonging to invasive species: *Pacifastacus leniusculus* and *Procambarus fallax f. virginalis.* The reflective marker is preferably reflective self-adhesive foil.

The advantages of the method for the ethological monitoring of crayfish and the system to implement the method based on this invention rest in particular in the elimination of any crayfish movement restrictions by the hardware components of the system, which thus causes no stress that would increase their cardiac activity so that the changes in cardiac activity only reflect the presence of undesirable pollutants in water subject to monitoring. Thanks to the method based on this invention, it is possible to acquire a very precise overview of the reactions of the monitored crayfish to a wide range of environmental changes allowing the impact of such changes on natural organisms living in the aquatic environment to be evaluated in a reliable manner.

### Explanation of drawings

The invention will be explained in detail by figures provided in the drawings where
- Fig. 1: shows a view of the crayfish with attached reflective markers,
- Fig. 2: shows a view of the system for the ethological monitoring of crayfish,
- Fig. 3: shows a diagram of the method for the ethological monitoring of crayfish,
- Fig. 4: shows a view of the delimited heart area,
- Fig. 5: shows a chart of the measured crayfish heart beat,
- Fig. 6: shows a chart of the measured heart beat using the method based on this invention compared to the ECG method.

### Examples of the preferred embodiments of the invention

It shall be understood that the specific cases of the invention embodiments described and depicted below are provided for illustration only and do not limit the invention to the examples provided here.

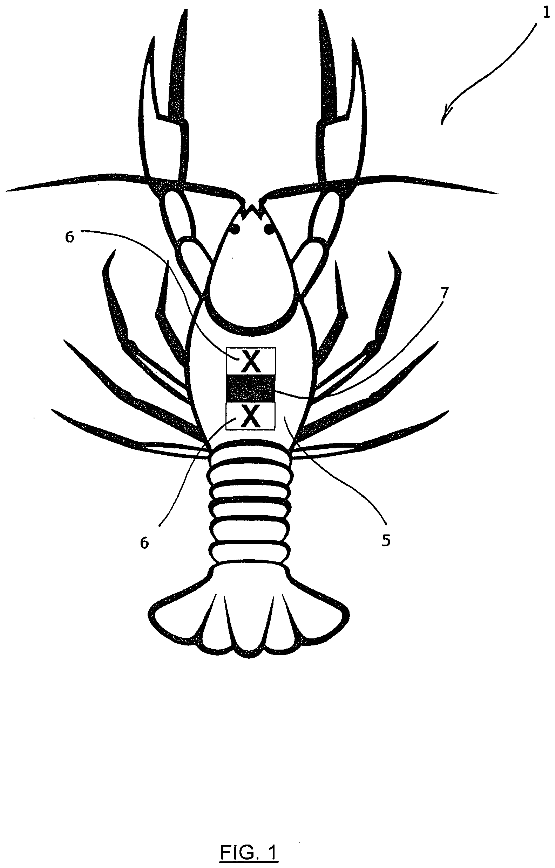

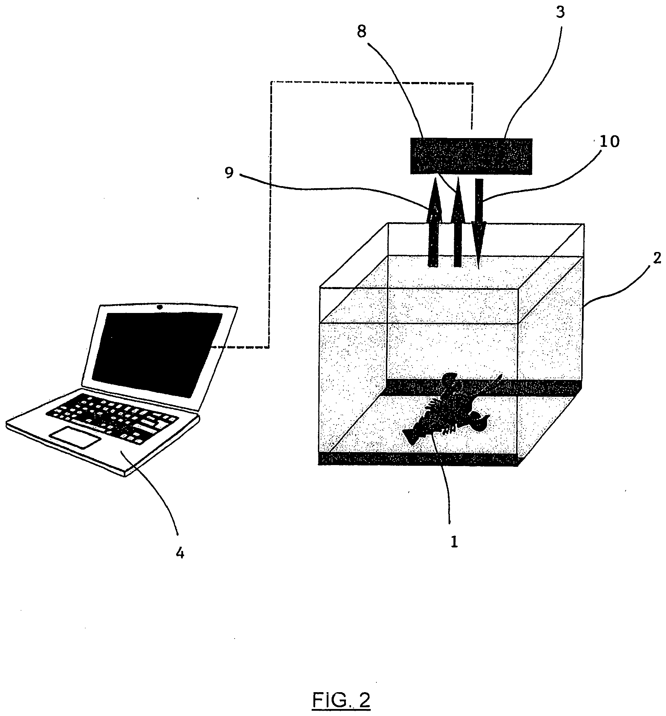

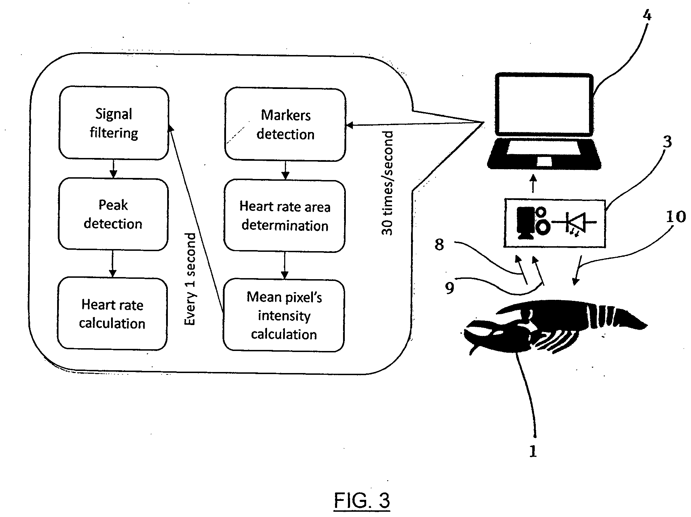

The system for the method for the ethological monitoring of crayfish **1** based on this invention called Kinect comprises an aquarium **2** of 20 × 30 × 20 cm in size, where three freely moving adult crayfish **1** of the invasive species *Pacifastacus leniusculus* and *Procambarus fallax f. virginalis* are placed and on whose carapaces **5** two reflective markers **6** delimiting the heart areas **7** of the crayfish **1** are affixed as illustrated in Fig. 1. However, the size of the aquarium **2** can be different in different examples of embodiments depending on the number of crayfish **1** placed in the aquarium **2.** The crayfish **1** can move freely in the aquarium **2** and the maximum possible speed of the crayfish **1** for the heart beat monitoring is 1.5 cm/s. This speed corresponds to the natural speed of the movement of the crayfish **1** in its natural environment. The crayfish **1** of the species *Pacifastacus leniusculus* can grow to the length of 115.0 mm and the weight of up to 56 g. The crayfish **1** of the species *Procambarus fallax f. virginalis* grows to the length of 50.5 mm and the weight of 6 g. In alternative examples crayfish **1** of alternative sizes and lengths can be used, which accentuates the universality of the system based on this invention. In other examples of the embodiment other species of crayfish **1** can be used. The universal nature of this method allows this method to be applied also to other species of crayfish **1.** Fig. 2 shows that the system is further comprised of an infra-red camera **3** and a computer **4.** The camera **3** consists of one distant source of infra-red radiation for directional emitting of infra-red radiation beams **10** at the delimited heart area **7** and the reflective markers **6,** and one receiver of infra-red radiation for simultaneous wireless reception of the beams **9** of the infra-red radiation reflected from the pericardium of the crayfish **1** within the delimited heart area **7** and the beams **8** of the infra-red radiation reflected from the reflective markers **6,** as illustrated in Fig. 3. The infra-red camera **3** is designed to acquire video recordings with the minimum frequency of 30 images per second. The infra-red camera **3** is localized at a distance of 600 mm above the aquarium to minimize the amount of beams reflected from the water surface. However, in another example of the embodiment, the camera **3** can be positioned also under the water surface of the aquarium **2.** The source on infra-red radiation in the infra-red camera **3** consists of three separated sources of infra-red radiation emitting three different wavelengths ranging from 827 to 850 nm with the power of 60 mW. The infra-red camera **3** has a resolution of 512 × 424 pixels, 12-bit depth and visual field of view 70.6 × 60 degrees.

The video recording of the reflected beams **8, 9** is transmitted from the infra-red camera **3** directly to the computer **4,** the next part of the system where this digitized data of the reflected beams **8, 9** is received. The computer **4** is equipped with a software module for the monitoring of the ethological parameters of the crayfish **1.** Based on the acquired data the software module can evaluate the data obtained from the beams **8** reflected from the reflective markers **6** placed on the carapaces **5** of the crayfish **1** and from the beams **9** reflected from the pericardium of the crayfish **1,** or, where applicable, the beams **9** reflected from the heart area 7 of the crayfish **1** delimited by the reflective markers **6** on the carapace **5** of the crayfish **1**, which means the determination of the position and directional orientation of the crayfish **1,** the position and directional orientation of the heart area **7,** heart beat and the trajectory of the crayfish **1** in real time.

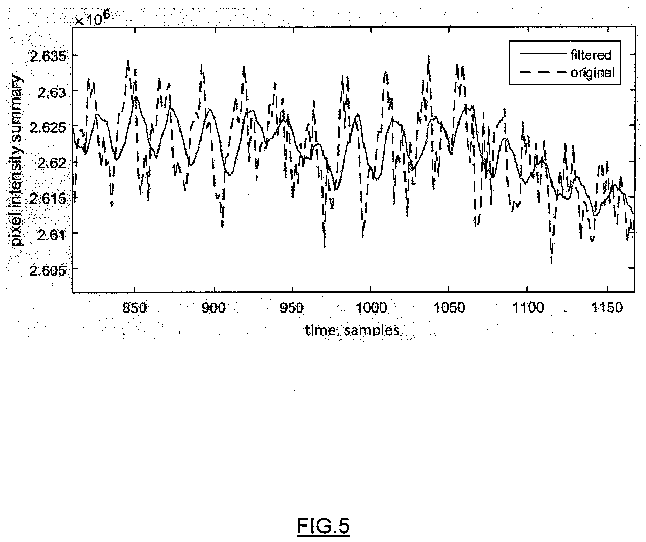

The data received by the computer **4** is in the form of a digital signal in the grey scale of the video recording being 512 × 424 pixels in size with the 12-bit depth. The intensity of each pixel corresponds to the infra-red radiation reflected from the objects exposed to the infra-red camera **3.** The first step for the successful ethological monitoring of the crayfish heart rate is a correct determination of the heart area **7.** This is achieved by the reflective markers **6** positioned on the carapace **5** of the crayfish **1**, which automatically delimit the heart area **7** of the crayfish **1**. The signal of the heart beat response is filtered using a noise-reduction method as illustrated in Fig. 5. Using methods for the detection of local extremes in signal, local minimums and maximums corresponding to the heart muscle contractions are determined. Based on the determination of the number of extremes per minute, the heart beat of the crayfish **1** is determined by this method. This method determined the heart beat in crayfish **1** of the species *Pacifastacus leniusculus* at 133 heart muscle contractions per minute, 128 heart muscle contractions per minute and 128 heart muscle contractions per minute. Accordingly, the system allows a contactless measurement of the heart beat of the crayfish **1** simultaneously with its trajectory and subsequent analysis to determine its physiological condition and changes in the quality of the aquatic environment. This system thus contributes to the minimization of stressful conditions for the crayfish **1,** as the crayfish **1** can freely move in the aquarium **2** without the presence of limiting fibres, conductors or sensors restricting the free movement of the crayfish 1 and causing stress as such, which is a parameter that can significantly affect the results of measurement in a negative manner.

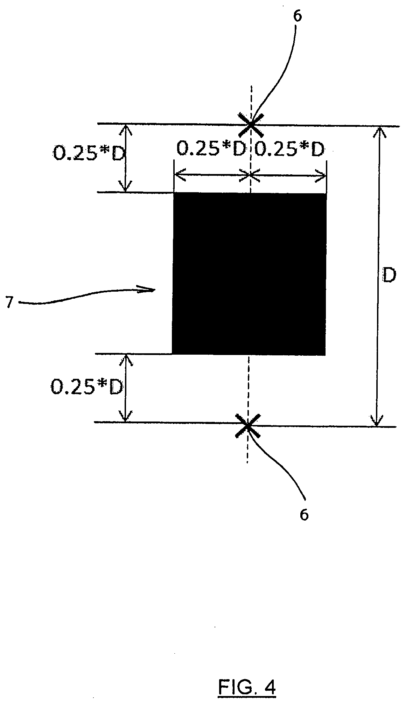

The reflective markers **6** are designed as reflective foils with high reflectance thanks to which the heart area **7** of the crayfish **1** is clearly and automatically delimited. The reflective foil reflects infra-red radiation and due to this fact it can be easily detected. Fig. 4 shows an illustration of the centres of the reflective markers **6,** which are then attached onto the carapace **5** of the crayfish **1.** The black rectangle between the reflective markers delimits the heart area **7** of the crayfish **1.** The size of the reflective marker **6** is determined by way of experiment for the follow-up detection using the infra-red camera **3** and it is 8 mm × 4 mm in size. The distance between the centres of the two reflective markers **6** is designated by a general parameter D and the distance of the rectangle of the heart area **7** from the centre of the reflective marker is 0.25*D. In this case the distance of the heart area **7** from the reflective marker **6** equals 2 mm.

The aquarium also includes a shelter for the crayfish **1.** The shelter may distort the data measured by the system based on this invention, and it may also result in the impossibility to carry out the measurement as the carapace **5** of the crayfish **1** cannot be exposed to infra-red radiation. A possible solution is to create the shelter from material transparent for infra-red radiation but opaque for visible light. Such materials include for example silicone- or polyethylene terephthalate-based fibres.

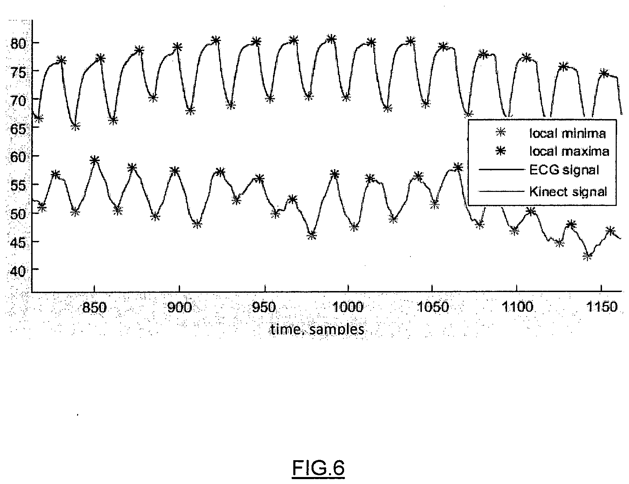

To compare the method for the ethological monitoring of crayfish 1 based on this invention, the conservative method for the ethological monitoring of crayfish **1** is used. It employs electrocardiography - ECG that is commonly used to determine the heart beat of crayfish **1** in water. This method uses electrodes introduced under the carapace **5** of the crayfish 1 up to the proximity of its heart. The electrodes are connected to fibres or cables allowing electrical signals of heart beat to be measured. The fibres are connected to an amplifier with the amplification factor 1,000. The amplifier is further connected to a converter from which the signal is transmitted to a software application with the sampling rate of 50 Hz. In this case the heart beat of the crayfish **1** is compared using the ECG method and the method based on this invention. The results of the experiment are illustrated in Fig. 6 where the upper curve represents the signal measured by the ECG method and the lower curve represents the signal measured by the method based on this invention - Kinect. As illustrated in Fig. 6, the local maximums and local minimums measured by the method based on this invention, which represent heart beat, correspond to the data measured by the commonly used ECG method. Using the method based on this invention, the heart beat of one species of the crayfish **1** was determined at 131.4 of heart beats per minute and using the ECG method at 131.7 heart beats per minute. Using the method based on this invention, the heart beat of the other species of the crayfish **1** was determined at 131.4 of heart beats per minute and using the ECG method at 125.4 heart beats per minute. The correlation coefficient between these two methods of the determination of the crayfish **1** heart beat is 0.95. Therefore, the method for the ethological monitoring of crayfish **1** based on this invention is comparable to the standard methods of ethological determination.

### Industrial applicability

The method for the ethological monitoring of crayfish and the system for the implementation of this method based on this invention can be used as bio-indicators for the laboratory or industrial monitoring of the pollution of water forming a natural environment of a wide range of aquatic organisms by various chemical pollutants.

### Overview of the positions used in the drawings

- 1: crayfish
- 2: aquarium
- 3: infra-red camera
- 4: computer
- 5: carapace of the crayfish
- 6: reflective marker
- 7: heart area of the crayfish
- 8: beam of infra-red radiation reflected from the reflective markers
- 9: beam of infra-red radiation reflected from the pericardium of the crayfish
- 10: beam of the emitted infra-red radiation

## Claims

1. A method for the real-time ethological monitoring of crayfish (1) in water, during which the crayfish (1) in an aquarium (2) is exposed to infra-red radiation beams (10) from a source of radiation designed to monitor the cardiac activity of the crayfish (1) and at the same time the crayfish movement is monitored by a camera (3) and measured data is transmitted to a computer (4) in the form of a digital signal which is processed in a software module designed to evaluate the reaction of the crayfish (1) to pollutants in water **characterized in that** the carapace (5) of the crayfish (1) is first equipped with at least two reflective markers (6) whose mutual distance delimits the heart area (7) of the crayfish (1), the aquarium (2), including the crayfish (1) is illuminated by the beams (10) of infra-red radiation and a distant receiver of infra-red radiation at the same time receives in a wireless manner the infra-red radiation beams (8) reflected from the reflective markers (6) and the infra-red radiation beams (9) reflected from the pericardium of the crayfish (1) within the delimited heart area (7), and the acquired data of the beams (8, 9) is transferred in the form of a digital signal of the video recording to a computer (4), where using a software module the position and directional orientation of the crayfish (1), the position and directional orientation of the delimited heart area (7), heart beat and the trajectory of the crayfish (1) movement is determined on a real-time basis.

2. The method according to claim 1, **characterized in that** an infra-red camera (3) is used as the distant source as well as the distant receiver of infra-red radiation.

3. The method according to claim 2, **characterized in that** the infra-red camera (3) is placed at a distance ranging from 400 mm to 1,500 mm from the bottom of the aquarium (2).

4. The method according to claim 2 or 3, **characterized in that** the video recording by the infra-red camera (3) is acquired with the minimum frequency of 30 images per second.

5. The method according to any of claims 1 through 4, **characterized in that** the reflective markers (6) are attached to the carapace (5) of the crayfish (1) of the invasive species: *Pacifastacus leniusculus* and *Procambarus fallax f. virginalis.*

6. A system to implement the method for the ethological monitoring of crayfish (1) in an aquarium (2) according to any of claims 1 through 5, **characterized in that** at least one crayfish (1) is equipped with at least two reflective markers (6) attached to the carapace (5) of the crayfish (1), whereby the mutual distance of the reflective markers (6) delimit the heart area (7) of the crayfish (1), and the system also includes at least one distant source of infra-red radiation to emit beams (10) of infra-red radiation at the aquarium (2), including the crayfish (1), at least one distant receiver of infra-red radiation for the wireless reception of the infra-red radiation beams (8) reflected from the reflective markers (6) and the infra-red radiation beams (9) reflected from the pericardium of the crayfish (1) within the delimited heart area (7), and at least one computer (4) for the reception of the digitized data of the beams (8, 9) in the form of video recordings equipped with a software module designed to determine the position and directional orientation of the crayfish (1), the position and directional orientation of the delimited heart area (7), heart beat and trajectory of the crayfish (1) on a real-time basis

7. The system according to claim 6, **characterized in that** the distant source and also the distant receiver of infra-red radiation is an infra-red camera (3) which is positioned above the aquarium (2) at a distance ranging from 400 mm to 1,500 mm from the bottom of the aquarium (2).

8. The system according to claim 6 or 7, **characterized in that** the infra-red camera (3) consists of the sources of infra-red radiation emitting different wavelengths ranging from 827 to 850 nm with the power of 60 mW.

9. The system according to any of claims 6 through 9, **characterized in that** the reflective marker (6) is attached to the carapace (5) of the crayfish (1) of the invasive species: *Pacifastacus leniusculus* and *Procambarus fallax f. virginalis.*

10. The system according to any of claims 6 through 10, **characterized in that** the reflective marker (6) is reflective foil with a self-adhesive layer.

## Patentansprüche

1. Die Verfahren der ethologischen Verfolgung der Krebse (1) im Wasser in der Echtzeit, bei welcher der Krebs (1) im Wasserbecken (2) der Wirkung der Infrarotstrahlen (10) aus der Strahlungsquelle für die Verfolgung der Herzaktivität des Krebses (1) ausgesetzt wird, und es wird gleichzeitig die Bewegung des Krebses durch die Kamera (3) überwacht, und die gemessenen Daten werden in den Computer (4) in der Form des Digitalsignals übertragen, welches durch das Softwaremodul zur Auswertung der Reaktionen des Krebses (1) auf Pollutanten im Wasser verarbeitet wird, **dadurch gekennzeichnet, dass** zur Schale (5) des Krebses (1) zuerst mindestens zwei Reflexionszeichen (6) befestigt werden, welche gegenüberliegend die Herzgegend (7) des Krebses (1) abgrenzen, das Wasserbecken (2) einschließlich des Krebses (1) wird durch die aus der Infrarotstrahlungsquelle emittierten Infrarotstrahlen (10) beleuchtet, wobei durch den Fernempfänger der Infrarotstrahlung gleichzeitig drahtlos die von Reflexionszeichen (6) reflektierten Infrarotstrahlen (8) und die vom Perikard des Krebses (1) in der abgegrenzten Herzgegend (7) reflektierten Infrarotstrahlen empfangen werden, und die erworbenen Daten der Strahlen (8, 9) werden in der Form des Digitalsignals der Bildaufnahme in den Computer (4) übertragen, wo mittels des Softwaremoduls die Lage und die Richtungsorientierung des Krebses (1), die Lage und die Richtungsorientierung der abgegrenzten Herzgegend (7), die Herzfrequenz und die Trajektorie der Bewegung des Krebses (1) in der Echtzeit festgelegt werden.

2. Verfahren nach dem Anspruch 1, **dadurch gekennzeichnet, dass** als Fernquelle sowie Fernempfänger der Infrarotstrahlung die Infrarotkamera (3) verwendet wird.

3. Verfahren nach dem Anspruch 2, **dadurch gekennzeichnet, dass** die Infrarotkamera (3) im Bereich ab 400 mm bis 1500 mm vom Boden des Wasserbeckens (2) platziert wird.

4. Verfahren nach dem Anspruch 2 oder 3, **dadurch gekennzeichnet, dass** die Bildaufnahme mit der Infrarotkamera (3) mit der minimalen Frequenz von 30 Bildern pro Sekunde aufgenommen wird.

5. Verfahren nach einem der Ansprüche 1 bis 4, **dadurch gekennzeichnet, dass** die Reflexionszeichen (6) zur Schale (5) des Krebses (1) aus der Gruppe der Invasions-Spezies: *Pacifastacus leniusculus* und *Procambarus fallax f. virginalis* befestigt wird.

6. System zur Durchführung der Art der ethologischen Verfolgung der Krebse (1) im Wasserbecken (2) nach einem der Ansprüche 1 bis 5, **dadurch gekennzeichnet, dass** mindestens ein Krebs (1) mit mindestens zwei zur Schale (5) des Krebses (1) befestigten Reflexionszeichen (6) versehen ist, wobei die Reflexionszeichen (6) gegenüberliegend die abgegrenzte Herzgegend (7) des Krebses (1) abgrenzen, und das System schließt weiter mindestens eine Fernquelle der Infrarotstrahlung zur Emittierung der Infrarotstrahlen (10) auf das Wasserbecken (2) einschließlich des Krebses (1), mindestens einen Fernempfänger der Infrarotstrahlung für den drahtlosen Empfang der von Reflexionszeichen (6) reflektierten Strahlen der Infrarotstrahlung und der vom Perikard des Krebses (1) in der abgegrenzten Herzgegend (7) reflektierten Infrarotstrahlen, und mindestens einen Computer (4) für den Empfang der digitalisierten Daten der Strahlen (8, 9) in der Form der durch das Softwaremodul aufgenommenen Bildaufnahme für die Festlegung der Lage und der Richtungsorientierung des Krebses (1), der Lage und der Richtungsorientierung der abgegrenzten Herzgegend (7), der Herzfrequenz und der Trajektorie des Krebses (1) in der Echtzeit ein.

7. System nach dem Anspruch 6, **dadurch gekennzeichnet, dass** die Fernquelle sowie der Fernempfänger der Infrarotstrahlung die Infrarotkamera (3) ist, welche oberhalb des Wasserbeckens (2) im Abstand ab 400 mm bis 1500 mm vom Boden des Wasserbecken (2) angeordnet ist.

8. System nach dem Anspruch 6 oder 7, **dadurch gekennzeichnet, dass** die Infrarotkamera (3) aus Quellen der Infrarotstrahlung besteht, welche unterschiedliche Wellenlängen im Bereich ab 827 bis 850 nm mit der Stärke von 60 mW produzieren.

9. System nach einem der Ansprüche 6 bis 9, **dadurch gekennzeichnet, dass** mit dem Reflexionszeichen (6) die Schale (5) des Krebses (1) aus der Gruppe der Invasions-Spezies: *Pacifastacus leniusculus* und *Procambarus fallax f. virginalis* versehen ist.

10. System nach einem der Ansprüche 6 bis 10, **dadurch gekennzeichnet, dass** das Reflexionszeichen (6) die Reflexionsfolie mit der selbstklebenden Schicht ist.

## Revendications

1. Procédé de contrôle en temps réel de l'éthologie des écrevisses (1) dans l'eau au cours duquel l'activité cardiaque de l'écrevisse (1) plongée dans un aquarium (2) et exposée à des rayons infrarouge (10) émis par une source de rayonnement est enregistrée, dans le même temps, les mouvements de l'écrevisse sont enregistrés à l'aide d'une caméra (3) et les données mesurées sont transmises à un ordinateur (4) sous la forme d'un signal numérisé utilisé par un module logiciel conçu pour évaluer les réactions de l'écrevisse (1) aux polluants présents dans l'eau, **caractérisé par le fait qu'**en premier lieu, au moins deux marqueurs réfléchissants (6) dont la position respective délimite la zone du coeur (7) de l'écrevisse (1) sont fixés sur la carapace (5) de l'écrevisse (1), puis l'aquarium (2) dans lequel est plongé l'écrevisse (1) est éclairé par les rayons (10) infrarouge émis par une source de rayonnement infrarouge, les rayons (8) infrarouge réfléchis par les marqueurs réfléchissants (6) et les rayons (9) infrarouge réfléchis par le péricarde de l'écrevisse (1) dans la zone délimitée du coeur (7) sont captés par un capteur de rayons infrarouge sans fil placé à distance et les données acquises des rayons (8, 9) sont transmises sous forme d'un signal digital d'enregistrement vidéo vers un ordinateur (4), qui détermine en temps réel grâce à un module logiciel, la position et l'orientation directionnelle de l'écrevisse (1), la position et l'orientation directionnelle de la zone délimitée du coeur (7), la fréquence cardiaque et la trajectoire du déplacement de l'écrevisse (1).

2. Le procédé selon la revendication 1 est **caractérisé par le fait qu'**une caméra infrarouge (3) est utilisée avec la source à distance ainsi que le capteur à distance des rayons infrarouge.

3. Le procédé selon la revendication 2 est **caractérisé par le fait que** la caméra infrarouge (3) est installée à une distance comprise entre 400 mm et 1500 mm du fond de l'aquarium (2).

4. Le procédé selon les revendications 2 ou 3 est **caractérisé par le fait que** la caméra infrarouge (3) enregistre les images à la fréquence minimum de 30 images par seconde.

5. Le procédé selon l'une des revendications 1 à 4 est **caractérisé par le fait que** les marqueurs réfléchissants (6) sont fixés sur la carapace (5) de l'écrevisse (1) des espèces invasives *Pacifastacus leniusculus* et *Procambarus fallax f. virginalis.*

6. Le système de réalisation du contrôle de l'éthologie des écrevisses (1) placées dans un aquarium (2) selon l'une des revendications 1 à 5 **est caractérisé par le fait qu'**au moins une écrevisse (1) est équipée d'au moins deux marqueurs réfléchissants (6) fixés sur la carapace (5) de l'écrevisse (1) et dont la position respective délimite la zone du coeur (7) de l'écrevisse (1), le système comprend également au moins une source d'émission de rayons (10) infrarouge placée à distance de l'aquarium (2) où est plongé l'écrevisse (1), au moins un capteur sans fil de rayons infrarouge placé à distance pour la réception des rayons (8) infrarouge réfléchis par les marqueurs réfléchissants (6) et les rayons (9) infrarouge réfléchis par le péricarde dans la zone du coeur délimitée (7) de l'écrevisse (1) et au moins un ordinateur (4) pour la réception des données numérisées des rayons (8, 9) sous forme d'un enregistrement vidéo et un module logiciel permettant de déterminer la position et l'orientation directionnelle de l'écrevisse (1), la position et l'orientation directionnelle de la zone délimitée du coeur (7), la fréquence cardiaque et la trajectoire de l'écrevisse (1) en temps réel.

7. Le système selon la revendication 6 est **caractérisé par le fait qu'**une caméra infrarouge (3) est utilisée avec la source à distance ainsi que le capteur à distance de rayons infrarouge. La caméra infrarouge (3) est installée à une distance comprise entre 400 mm et 1500 mm du fond de l'aquarium (2).

8. Le système selon les revendications 6 ou 7 est **caractérisé par le fait que** la caméra infrarouge (3) enregistre les rayons infrarouge émis par les sources de rayonnement d'infrarouge de diverses longueurs d'ondes comprises entre 827 à 850 nm et d'une puissance de 60 mW.

9. Le système selon l'une des revendications 6 à 9 est **caractérisé par le fait que** les marqueurs réfléchissants (6) sont installés sur la carapace (5) des écrevisses (1) d'espèces invasives *Pacifastacus leniusculus* et *Procambarus fallax f. virginalis.*

10. Le système selon l'une des revendications 6 à 10 est **caractérisé par le fait que** les marqueurs réfléchissants (6) sont constitués d'une feuille réfléchissante revêtue d'une couche autocollante.
